# EUROPEAN PATENT APPLICATION

(11) **EP 0 835 660 A1**
(43) Date of publication of application: **15.04.1998**
(21) Application number: 96870130.0
(22) Date of filing: 14.10.1996
(51) Int. Cl.: A61K 31/70

(54) **Products containing methylcobalamin for the treatment of multiple sclerosis or other demyelinating conditions**

(71) Applicant: Merckx, Gaston Edmond Filomena, 2650 Edegem (BE)
(72) Inventor: Merckx, Gaston Edmond Filomena, 2650 Edegem (BE)
(74) Representative: Van Reet, Joseph

(57) **Abstract**

Products containing methylcobalamin in an overdose corresponding to a weekly intramuscular dose higher than 1000 micrograms, preferably higher than 1200 micrograms and corresponding more preferably to a weekly intramuscular dose of at least 15OO micrograms, and an antidepressant enhancing the serotonin level as a combined preparation for simultaneous, separate or sequential use in the treatment of multiple sclerosis or other demyelinating conditions.

## Description

The present invention relates to products containing methylcobalamin for the treatment of multiple sclerosis (MS) or other demyelinating conditions.

Multiple sclerosis is a common neurological disorder characterised either by episodic patches of inflammation and demyelination (relapsing remitting evolution), or by a chronic progressive evolution, which can occur anywhere in the central nervous system (CNS) almost always without any involvement in the peripheral nerves.

The pathogenesis of multiple sclerosis has not yet been elucidated. Some believe that the pathogenesis involves local disruption of the blood brain barrier, a local immune and inflammatory response, with subsequent damage to myelin and hence to neurons. Others suggest that an important aspect of the pathogenesis of multiple sclerosis is a possible disturbance in vitamin B12 (cobalamin) metabolism (see Reynolds EH, Linnell JC, Faludy JE - Multiple sclerosis associated with vitamin B12 deficiency. Arch. Neurol. 48: 808, 1991 and Reynolds EH, Bottiglieri T, Laundy M, Crellin RF, Kirfer SG. Vitamin B12 metabolism in multiple sclerosis. Arch. Neurol. 49:649, 1992).

Based on this suggestion, Kira J. et al (see Kira J, Tobimatsu S, Goto I. Vitamin B12 metabolism and massive-dose methyl vitamin B12 therapy in Japanese patients with multiple sclerosis. Internal Medicine Vol. 33, No. 2, Febr. 1994), have examined the effects of high methyl vitamin B12 overdoses on motor disability and multimodality evoked potential (MEP) abnormalities in six patients with chronic progressive MS. After a treatment for 6 months with oral daily doses of 60 mg, it could be concluded that the MEP abnormalities reflecting the demyelinating lesions in the afferent pathways were improved but the motor disability did not improve clinically. A massive-dose methyl vitamin B12 therapy was therefore recommended as a possible adjunctive therapy to immunosuppressive treatments for multiple sclerosis.

The present inventor has also found that a therapy with vitamin B12 overdoses has a positive effect on the reflexes of an MS patient indicating the occurrence of myelin reconstruction. This effect could be achieved with lower overdoses than the overdoses by Kira et al, for example with intramuscular (IM) doses of about 1500 µg per week. However, even at these lower doses, strong side-effects of the vitamin B12 therapy were observed comprising pains distributed over the entire body, including pains in the feet, legs, shoulders, ears, teeth, etc. These pains occurred when administering hydroxocobalamin but extended and increased considerably when changing over to methylcobalamin administration. Further, the motor ability did not improve by the vitamin B12 therapy, moreover, spasticity occasionally turned over in weakness and buckling reactions in the legs.

An object of the present invention is therefore to provide a treatment of MS whereby not only the myelin reconstructing effects of methylcobalamin overdoses can be improved but whereby the motor ability can further be achieved and wherein especially the observed side-effects of methylcobalamin overdoses can be remedied.

According to the invention, these objects can be reached by combining the methylcobalamin overdoses with a simultaneous, separate or sequential administration of an antidepressant enhancing the serotonin level.

The use of antidepressants which work by interfering with the inactivation of neurotransmitters, in particular noradrenaline and/or serotonin, for the treatment of multiple sclerosis or other demyelinating conditions has already been disclosed in WO96/11OO9. In this international patent application, these antidepressants are used, in combination with precursors of said neurotransmitters, for compensating for the effects of nerve damage caused by MS or the other demyelinating conditions. Such a possible compensation of nerve damage has for example been observed by Scott et al. (Scott, CF Jr., Cashman N, Spitler LE. Experimental Allergic Encephalitis ; Treatment with Drugs which alters CNS Serotonin Levels. Journal of Immunopharmacology, 4(3), 153-162 (1982-83)) in experimental allergic encephalitis (EAE) of Guinea pigs, which is an acute autoimmune demyelinating disease caused artifically by immunization with Guinea pig myelin basic protein (GPBP). They found that this disease may more particularly be due to an immune response directed against CNS serotonin receptors and that the effect of such a serotonin receptor blockage could be compensated for by drugs, such as imipramine and tryptophan, which enhance CNS serotonin levels. If a serotonin receptor blockage could be found in multiple sclerosis, such a therapy could also be effective for the treatment of MS.

In WO 91/11009, it is further suggested to give a background course of vitamin B12, more particularly cyanocobalamin or hydroxocobalamin, in daily amounts which may be for example the conventional daily requirement for this vitamin. In the treatment examples, it was desirable for patients with chronic progressive MS, to include an 8-10 week course of 1000 micrograms of hydroxocobalamin (intra-muscular) per week at the start of the treatment with the antidepressant and then 1000 micrograms every ten days thereafter. Since it is generally known that a vitamin B12 deficiency leads to demyelination of the nerves, this optional hydroxocobalamin background course was clearly intended to have a positive effect on myelin reconstruction.

The treatment proposed by the present invention is, on the contrary, first of all based on methylcobalamin overdoses effective for achieving myelin reconstruction and thus for remedying nerve damage. Indeed, methylcobalamin appeared according to Kira et al (see supra) to be much more effective for myelin reconstruction than hydroxocobalamin. As already mentioned hereinabove, the side-effects observed by the inventor are however also much more pronounced for methylcobalamin than for hydroxocobalamin. According to the present invention, it has now been found surprisingly that a particular group of the antidepressants, which have been suggested in WO96/11009 for compensating for the effects of nerve damage caused by MS or other demyelinating conditions, was also very effective in remedying the side-effects of methylcobalamin overdoses, although such overdoses do not lead to nerve damage but, on the contrary, to nerve repair.

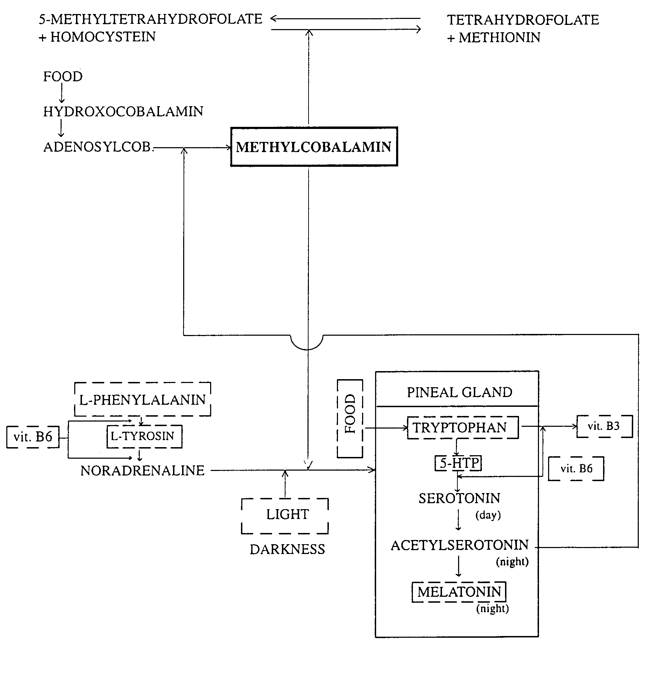

This surprising effect can be explained on the basis of a general hypothesis developed by the present inventor with respect to the etiology of multiple sclerosis and the observed side-effects of methylcobalamin overdoses. This hypothesis is based on a number of biochemical working mechanisms which are already known per se and which are schematically indicated on the scheme of the general hypothesis shown in the sole figure, some correlations and interactions between these separated systems being described for the first time by the present inventor.

### 1. Vitamin B12 metabolism

Vitamin B12 is delivered by the food mainly in the form of hydroxocobalamin. This hydroxocobalamin is transferred to the cells through the intermediary of several transcobalamins and is taken up by these cells, in particular by the cells of the CNS. In these cells, hydroxocobalamin is converted into adenosylcobalamin and further to methylcobalamin, involving a reduction of the central cobalt atom of the cobalamin (see Parry TE. Megaloblastic anaemia in the elderly. Baillière's Clinical Haematology - Vol. 1, No. 2, June 1987). In multiple sclerosis the transfer and uptake of hydroxocobalamin is suspected to be disturbed by genetic errors.

### 2. Transmethylation reaction

The transmethylation reaction of 5-methyltetrahydrofolate and homocystein produces tetrahydrofolate and methionine which have a basic effect on myelin reconstruction. The interaction with the myelin reconstruction processes is not yet completely understood but it is believed that a major part is due to the influence of tetrahydrofolate and/or methionin on the DNA-synthesis for myelin reconstruction (see Hoffbrand AV. Role of Vitamin B12 in DNA synthesis. International Symposium "Thomas Addison and his Diseases : 200 Years on". London - Padua, May 1993).

The presence of methylcobalamin is essential for the transmethylation reaction in that it catalyses this reaction. An overdose of vitamin B12, in particular of methylcobalamin as suggested by Kira et al (see supra), is thus required for achieving myelin reconstruction.

### 3. Biological clock function of the pineal gland

In the pineal gland (being one of the important human biological clocks), tryptophan is converted, on the one hand, into vitamin B3 and, on the other hand, into 5-hydroxotryptophan (5-HTP), which is further converted into serotonin. At night, the serotonin is converted into acetylserotonin and further into melatonin. The serotonin produced by day is an important activity, mobility and good mood promoter and immuno-protector whilst melatonin produced at night is an important recovery and protection hormone responsible for cell protection and regeneration and also for immuno-regulation. The working of the pineal gland biological clock is controlled by noradrenaline released at night by sympathic nerve endings and acting on β-adrenergic receptors of the pineal gland (see Ozawa K, Segawa T. Methyl B12 and the intracerebral signal transduction system. Methyl B12 Forum, "Methyl B12", Internet K.K., Tokyo, 1993, pp.20-23). Ozawa et al. further explained the effects of methylcobalamin, which was known to be efficacious in the treatment of sleep-wake rhythm disorders, on the working of the biological clock as being based on an acceleration or an improval of signal transmissions via β-receptors. So a lack of methylcobalamin leads to a hypo-sensitivity of the pineal gland and so to a dysfunction of the biological clock.

An analogous explanation was given by Honma et al (Honma K, Kohsaka M, Fukuda N, Morita M and Honma S. Effects of vitamin B12 on plasma melatonin rhythm in humans : increased light sensitivity phase-advances the circadian clock ? Experientia 48 (1992), Birkhäuser Verlag, CH-4010 Basel/Switzerland) for the phase-advances of the biological clock they observed as a result of oral methylcobalamin administrations of 3 x 1 mg/day for 4 weeks. These effects were more particularly explained as being the result of an increased light sensitivity due to an increased plasma level of methylcobalamin. The test persons subjected to the methylcobalamin trial had indeed a mean total cobalamins plasma level at the end of the trial of about 1047 pg/ml (which is still only a limited overconcentration) whilst the test persons subjected to the placebo trial had a mean plasma level of only about 511 pg/ml.

According to the present inventor, the level of methylcobalamin has not only an effect on the biological clock function of the pineal gland but a disturbance of this function has in its turn a negative effect on the vitamin B12 metabolism. The appearance of multiple sclerosis can thus be explained as being basically and first of all based on a disturbed transfer to and uptake of hydroxocobalamin by the CNS cells, and may especially be initiated by additional specific circumstances, such as a disturbed input of vitamin B12 through the food caused for example by pregnancy, presence of a broad fish tapeworm, food composition, lack of the intrinsic factor required for vitamin B12 uptake in the stomach, shock or stress situations, etc., aside of a disregulation of the pineal gland biological clock i.a. as a result of the methylcobalamin deficiency. A disregulation of the biological clock results indeed in a reduced cell protection and regeneration, and so also in a reduced protection of methylcobalamin against oxidation. This may explain for example the fact that in areas farther away from the equator there are more MS patients than is in areas closer to the equator, even within a same country (as Great Britain), since a less favourable geographical situation may lead to a less favourable function of the biological clock. An increasing distance to the equator indeed involves an increasing unequality between day and night periods and a more reduced daylight intensity. The appearance of multiple sclerosis is in other words, according to this hypothesis, based on an auto-destructive mechanism starting from an intracellular methylcobalamin deficiency leading to myelin deconstruction and to a reduced working of the biological clock and so to a reduced immunity, cell protection and regeneration and in particular to a loss of protection of the methylcobalamin. Methylcobalamin is indeed very sensitive to oxidation processes and oxidizes for example already when being exposed to light. Further, a disregulation of the biological clock may, due to the resulting decreased serotonin level, also lead to a loss of protection of the myelin basic protein itself since according to Field et al. (Field EJ, Gaspary EA, and Carnegie PR. Lymphocyte sensitization to basic protein of brain in malignant neoplasia; experiments with serotonin and related compounds. Nature 233:284, 1971), serotonin inhibits a lymphocyte response to myelin basic protein. On this basis, the immuno-reactions, seen in MS patients and having led to the development of drugs such as β-interferon, find their explanation not as being primary causes however, but as being secondary reactions.

In view of the vitamin B12 deficiency in the cells and in view of the disturbed vitamin B12 metabolism, the treatment of multiple sclerosis comprises according to the present invention first of all the administration of methylcobalamin overdoses, corresponding more particularly to weekly intramuscular doses higher than 1000 micrograms, preferably higher than 1200 micrograms and corresponding most preferably to weekly intramuscular doses of at least about 1500 micrograms . The methylcobalamin can for example also be administered in oral dosage forms containing about ten times the above indicated amounts in view of the lower absorption efficiency of oral dosage forms. As explained hereinabove, such high methylcobalamin doses are required to obtain myelin reconstruction.

On the other hand, the negative side-effects of such high methylcobalamin doses observed by the inventor, are attributed by him to another - and enhanced - disregulation of the same biological clock. Indeed, the present inventor has found that not only a too low methylcobalamin level may cause a hypo-sensitivity and so a disregulation of the biological clock but also high methylcobalamin levels, for example total cobalamin plasma levels of about 20,000 pg/ml reached after a continued therapy with the methylcobalamin overdoses according to the invention, leading to a hyper-sensitivity of the pineal gland mechanism and so to a still enhanced disturbance of the biological clock effects and to depressed serotonin and melatonin levels. An enhanced depression of the serotonin level leads not only to an enhanced lack of neurotransmission, indispensable for action, movement, mobility, good mood, etc., but also to a reduced protection against auto-destructing reactions in which attacks of myelin basic protein are elicited by T-lymphocytes. On the other hand, an enhanced depression of the melatonin level leads to an enhanced loss of cell protection and recovery and of immuno-protection. The basic idea of the present invention consists in that the side-effects of methylcobalamin overdoses required for obtaining myelin reconstruction can be obviated by compensating for the enhanced disregulation of the biological clock function of the pineal gland due to the methylcobalamin overdoses, more particularly by means of antidepressants enhancing the serotonin level in the CNS.

In view of the hereinabove described treatment of multiple sclerosis or other demyelinating diseases, the products according to the invention contain methylcobalamin in the described overdose and an antidepressant enhancing the serotonin level as a combined preparation for simultaneous, separate or sequential use in these diseases. The two compounds may be administered in the same dosage form, or may be in separate dosage forms in a same pack or in separate packs for administration at separate times but so as to be effective together in the body.

Dosage forms containing the two compounds, and possible further active ingredients to be described hereinafter, or the separate dosage forms may be for example in the form of baxters, ampoules for intravenous or intramuscular injections, capsules, tablets, lozenges, softgels, creams, etc.

The antidepressant enhancing the CNS serotonin level may be either a monoamine oxidase inhibitor and/or a serotonin uptake inhibitor, preference being given to monoamine oxidase inhibitors and in particular to monoamine oxidase A inhibitors. Indeed, monoamine oxidase inhibitors do not only enhance the serotonin level but also the noradrenaline level thus enhancing the melatonin producing function of the pineal gland. Monoamine oxidase A inhibitors are especially preferred since they still allow the monoamine oxidases B to decompose monoamines such as tyramines which might otherwise possibly accumulate for example when eating food, such as aged cheese, with a high tyramine (or tyrosine) content. Tyrosine and also phenylalanine are indeed precursors of noradrenaline as shown in the figure. A certain minimum amount is of course always required for producing the necessary amount of noradrenaline.

The antidepressant enhancing the serotonin level can be selected from the group comprising antidepressants acting exclusively or mainly on the serotonin level such as fluvoxamine, trazodone, ifoxetine, fluoxetine, citalopram and CGP 6085 A ; antidepressants acting preferably on the serotonin level such as clomipramine and clanopramine and antidepressants acting both on the serotonin and the noradrenaline level such as imipramine, amitriptyline, doxepine, dosulepine, nortriptyline, clovoxamine, phenelzine, iproniazid, nialamide, isocarboxazide, iproclozide, brofaromine, moclobemide, quinupramine, butriptyline, trimipramine, melitracene, opipramol, a particular preference being given to the antidepressant moclobemide. This latter class or type of antidepressants is preferred for the reason that they enhance not only the serotonin level but also the noradrenaline level thus enhancing the melatonin producing function of the pineal gland. A particular preference is given to moclobemide in quantities of up to for example 900 milligrams daily. The noradrenalin level enhancing effect can also be achieved by means of noradrenaline precursors, more particularly by means of L-phenylalanin and tyrosin. In a preferred embodiment, such a noradrenaline precursor is also included in the products according to the invention.

The products according to the invention preferably also contain a serotonin precursor, in particular L-tryptophan and/or 5-hydroxotryptophan (5-HTP), 5-HTP being strongly preferred in view of the fact that it crosses the blood brain barrier more effectively than tryptophan and that is also the immediate precursor of serotonin. After an initial period of at least 3 to 4 weeks, it might be possible for some MS patients to omit the antidepressant in case a sufficient serotonin production is realised by the pineal gland due to the administrations of the serotonin precursor, in particular 5-HTP.

In a further preferred embodiment of the products according to the invention, they contain moreover niacin and/or niacinamid (vitamin B3). By increasing the vitamin B3 level in the CNS, in particular in the pineal gland, the reaction from tryptophan into vitamin B3 is counteracted thus enhancing the reaction from tryptophan into 5-hydroxotryptophan and further to serotonin. This latter reaction is catalyzed by vitamin B6 (pyridoxin) which may advantageously also be included in the products according to the invention. Vitamin B6 further catalyses the reaction from phenylalanin to tyrosin and further to noradrenaline which reactions are important for getting a better stimulation of the working of the pineal gland as explained hereinabove, i.e. for stimulating its biological clock effects.

The administration of the products according to the invention is preferably accompanied by light therapy and this preferably in the morning and/or forenoon. Such a light therapy is also intended to enhance the biological clock effects of the pineal gland, more particularly its serotonin (and further on its melatonin) producing function.

Preferably, the products according to the invention additionally contain melatonin which is advantageously administered 1 to 2 hours before sleeping time.

Typical amounts of the above described active ingredients are for :
- methylcobalamin :
   - intramuscular injections of 3 x 500 µg weekly, and during the first month treatment for example 3 x 1000 µg weekly, or
   - oral administrations of up to 20 x 500 µg daily in tablets
- moclobemide : up to 900 mg daily
- other antidepressants : as prescribed therefor for their antidepressant effects
- 5-hydroxotryptophan : for example up to 1000 mg daily
- L-phenylalanin and/or L-tyrosin : up to 600 mg daily
- niacin or niacinamid (vitamin B3) : up to about 1500 mg daily
- pyridoxin (vitamin B6) : up to 300 mg daily
- melatonin : up to 10 mg daily

### Case history

A 56 years old female having chronic progressive MS for about 13 years. Eight years ago, she had a broad fish tapeworm (which consumes the vitamin B12 present in the human intestines) and a vitamin B12 therapy was started, more particularly monthly intramuscular injections of 5000 µg hydroxocobalamin. After a few weeks better reflexes were observed by also the appearance of pain effects distributed over her body,i.e. from their feeth, legs, hips, shoulders to their teeth.

Two years later, the intracellular cobalamin level was determined showing an absolute intracellular total cobalamin and methylcobalamin deficiency notwithstanding the high serum level of about 20,000 pg/ml. Raising of the hydroxocobalamin administration to 3 x 1000 µg weekly for 2 years resulted in better cellular levels but the methylcomponent was still deficient.

About 4 years ago, a treatment with weekly injections of 3 x 500 µg methylcobalamin was started. Again better reflexes were observed but the pains accompanying the vitamin B12 therapy were markedly worse when changing over from hydroxocobalamin to methylcobalamin. Last year a light therapy in the morning and a melatonin dosage in the evening was started without much effect on the observed side-effects of the methylcobalamin therapy. During six months, the IM injections were replaced by the daily intake of 20 x 500 µg tablets of methylcobalamine without notable effects.

Recently, a moclobemide therapy was started in a quantity of 400 mg daily. Already some days after starting this therapy, the first signs of recovery could be observed, including a reduction of the pains, a better balance, a sharper mental reflex, an improved bladder function and a loss of obstipation, a rearrangement of both blood pressure and pulse frequency and an improved mobility with a disappearance of the buckling effects in the legs. Before this treatment, the patient was more particularly unable to walk autonomously, the balance being completely disturbed, and presenting all symptoms mentioned hereabove. Further on, all these ameliorations are keeping on and grow in a slow but stable and undeniable way. Meanwhile 5-HTP is added to the therapy.

## Claims

1. Products containing methylcobalamin in an overdose corresponding to a weekly intramuscular dose higher than 1000 micrograms, preferably higher than 1200 micrograms and corresponding more preferably to a weekly intramuscular dose of at least 15OO micrograms, and an antidepressant enhancing the serotonin level as a combined preparation for simultaneous, separate or sequential use in the treatment of multiple sclerosis or other demyelinating conditions.

2. Products according to claim 1, wherein said antidepressant is a monoamine oxidase inhibitor and/or a serotonin uptake inhibitor.

3. Products according to claim 2, wherein said antidepressant is a monoamine oxidase inhibitor, preferably a monoamine oxidase A inhibitor.

4. Products according to claim 1, wherein said antidepressant is selected from the group consisting of fluvoxamine, trazodone, ifoxetine, fluoxetine, citalopram, CGP 6085 A, clomipramine, clanopramine, imipramine, amitriptyline, doxepine, dosulepine, nortriptyline, clovoxamine, phenelzine, iproniazid, nialamide, isocarboxazide, iproclozide, brofaromine, moclobemide, quinupramine, butriptyline, trimipramine, melitracene, opipramol, a particular preference being given to the antidepressant moclobemide.

5. Products according to claim 4, wherein said antidepressant is selected from the group consisting of imipramine, amitriptyline, doxepine, dosulepine, nortriptyline, clovoxamine, phenelzine, iproniazid, nialamide, isocarboxazide, iproclozide, brofaromine, moclobemide, quinupramine, butriptyline, trimipramine, melitracene, opipramol, a particular preference being given to the antidepressant moclobemide.

6. Products according to any one of the claims 1 to 5, containing further a serotonin precursor, in particular L-tryptophan and/or 5-hydroxotryptophan.

7. Products according to claim 6, wherein said serotonin precursor comprises 5-hydroxotryptophan.

8. Products according to claim 6 or 7, wherein said antidepressant is omitted after an initial treatment period of at least three to four weeks.

9. Products according to any one of the claims 1 to 8, containing further niacin and/or niacinamid (vitamin B3).

10. Products according to any one of the claims 1 to 9, containing further pyridoxin (vitamin B6).

11. Products according to any one of the claims 1 to 10, containing further melatonin.

12. Products according to any one of the claims 1 to 11, containing further a noradrenaline precursor, in particular phenylalanin and/or tyrosin.
